# EUROPEAN PATENT SPECIFICATION

(11) **EP 0 415 504 B1**
(45) Date of publication and mention of the grant of the patent: **28.07.1993**
(21) Application number: 90202314.2
(22) Date of filing: 29.08.1990
(51) Int. Cl.: A61M 36/04

(54) **Implanter**
Implantiervorrichtung
Dispositif d'implantation

(30) Priority: 30.08.1989 NL 8902186
(43) Date of publication of application: 06.03.1991
(73) Proprietor: N.V. Nederlandsche Apparatenfabriek NEDAP, NL-7141 DE Groenlo (NL)
(72) Inventor: De Jong, Hendrik Johannes, NL-7141 XJ Groenlo (NL)
(74) Representative: Smulders, Theodorus A.H.J., Ir.

(56) References cited:
- US-A- 3 430 626
- US-A- 3 744 493
- US-A- 4 263 910
- US-A- 4 820 267

## Description

The invention relates to an implanter for implanting an implant in an animate being, comprising a housing mounting one end of a hollow injection needle, spring means inside the housing and further a plunger adapted to extend into the hollow needle from the end of the hollow injection needle that is turned towards the housing.

Such an implanter is known from US Patent No. 3,744,493 and can be used for implanting via the hollow injection needle a usually cylindrical implant, containing for instance a medicinal preparation or an electronic circuit for remote detection, in animate beings, such as cattle.

A drawback of the known implanter is that two different operations are required for introducing the injection needle and subsequently introducing the implant into the body of an animate being via the injection needle. First the injection needle is stuck into the body and then the implant is introduced into the body via the injection needle by means of a plunger. On the one hand, this takes relatively much time, which may pose a problem, an animal during treatment being somewhat excited as it is and hence being capable of jerky movements. Moreover there is a real chance that the person who carries out the implantation, for the very reason of carrying out the operation as swiftly as possible, retracts the needle too soon, i.e. before the needle has been introduced deep enough, or that the animal recoils somewhat precisely during the operation. The depth at which the implant is lodged in the body may then be undesirably small, there being a chance the animal will lose the implant. This is particularly important when the implant contains an identification chip which is to remain in the body for a long time.

Further, US Patent No. 4,820,267 discloses an implanter which comprises a plunger fixedly mounted in the housing and a hollow injection needle movable relatively to the plunger. After having been introduced into the body of for instance an animal, the injection needle is manually retracted into the housing with the fixed plunger retaining an implant disposed in the injection needle, so that it remains behind in the body. This known device, too, involves the risk of the needle being retracted too soon, as a result of which the implant is not properly introduced.

The invention aims to overcome the drawbacks outlined hereinabove and generally to provide an implanter that is easy to operate but functions reliably.

To this effect an implanter of the type described hereinabove is characterized according to the invention by locking means for retaining the plunger against the pressure of the spring means in a position where the plunger extends into the hollow needle over a predetermined distance, said locking means comprising unlocking means which abut the body of the animate being when the injection needle has been introduced into the body to a predetermined depth and subsequently automatically unlock the locking means, causing the plunger to be pressed by said spring means against an implant disposed in operation in the injection needle for retaining the implant when the injection needle is being retracted from the body.

It is observed that US Patent No. 3,430,626 discloses a device for injecting fluids into cattle by means of an injection needle, the device comprising bar-shaped driving means which in operation force the fluid to be injected into the injection needle but which are normally arrested in an inoperative position. The device further comprises a feeler mounted with a spring and extending beyond the tip of the injection needle, which upon introduction of the injection needle into the body of an animal moves in a rearward direction relatively to the injection needle and thus unlocks the driving means. Under the influence of the force exerted by a coil spring the driving means then force the fluid to be injected into the injection needle.

In this known device the driving means are unlocked when the injection needle has only barely, if at all, penetrated the animal's skin. Moreover, the driving means are designed to actually force the fluid to be injected through the injection needle introduced into the body, which takes some time. Accordingly, in this known device, too, there is a real chance that the injection needle is retracted too soon and the injection operation will not have the desired result.

The invention will now be further described with reference to the accompanying drawings of some embodiments, in which:
Fig. 1 is a schematic longitudinal sectional view of an embodiment of an implanter according to the invention;
Figs. 2-4 schematically show in what way an implant can be introduced into a body using an implanter according to the invention;
Fig. 5 shows a holder for implants,to be used with an implanter according to the invention;
Fig. 6 schematically illustrates the loading of an implanter according to the invention using a holder according to Fig. 5; and
Fig. 7 is a schematic longitudinal sectional view of another embodiment of an implanter according to the invention.

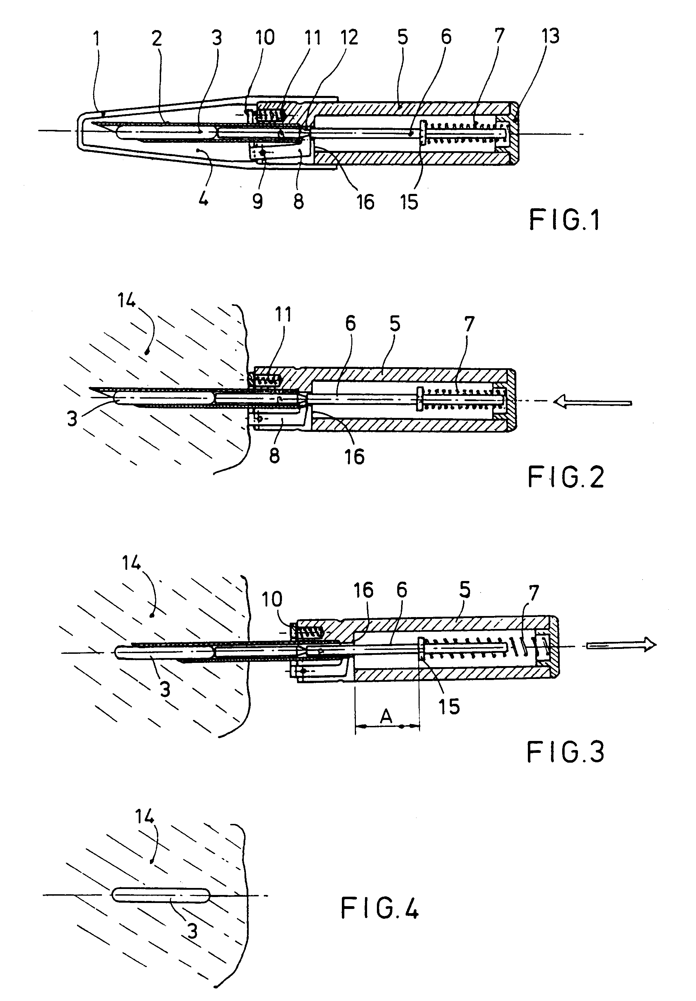

Fig. 1 schematically shows a longitudinal sectional view of a first embodiment of an implanter according to the invention. The implanter shown comprises a hollow needle 2, which is preferably protected by a detachable cap 1 when the implanter is not being used.

An implant 3 has already been provided in the needle 2. In the space 4 a disinfectant fluid or paste may be provided to prevent wound infections after the implantation.

The needle 2 can be mounted on a housing 5 and is optionally mounted for rapid detachment, for instance by means of a bayonet catch. In the embodiment shown the housing has a substantially cylindrical form and is hollow. Provided in the housing is a plunger 6 which extends into the hollow needle over a predetermined distance and can be moved to the left, i.e. into the needle, by means of a pressure spring 7 pressing against a shoulder 15 of the plunger, but which plunger is retained by locking member 8 adapted to pivot about a pin 9. Disposed at the end of the housing which is turned towards the needle 2 is a pressure plate 10 which, in this embodiment, forms one whole with a lock 8 and (as shown) is pressed to the left by a spring 11, thus pressing lock 8 into a groove 12 of the plunger 6. Further, the housing 5 is closed by a cover 13 supporting the spring 7.

Fig. 2 shows the situation where the needle 2 has been introduced into the body 14 to the proper depth. The lock 8 now releases the plunger 6 due to the pressure plate 10 pressing against body 14, the spring 11 thus being compressed and the lock pivoting about the pin 9.

During the subsequent retractive motion the plunger 6 continue to retain the implant 3 in its position within the body owing to the expansion of spring 7 (Fig. 3). Distance A between the shoulder 15 and the inner end wall 16 of the housing proximal to the needle diminishes and will be = 0 at the moment when the implant is no longer surrounded by the needle 2 and thus will of itself remain behind in the body (Fig. 4). Thus the implanting operation has been completed swiftly and without fault. The implanter can now be disposed of or be prepared for reuse. An important advantage of an implanter according to the invention is that the plunger 6 is automatically actuated as soon as the needle has been introduced into the body to sufficient depth. Moreover, the plunger only has to retain the implant during the retraction of the needle, but need not actively force the implant from the needle. Implantation of the implant at insufficient depth is prevented and also during the implantation no further operations are necessary in addition to introducing and retracting the needle.

The implanter can be prepared for reuse by bringing plunger 6 into the initial position by means of a bar-shaped auxiliary device, after which a new implant 3 can be provided in needle 2. Clearly, this must be effected aseptically and new disinfectant fluid or paste must be provided. To facilitate the above operation and to ensure asepsis, according to the invention a special holder can be used which may contain a plurality of implants and disinfectants.

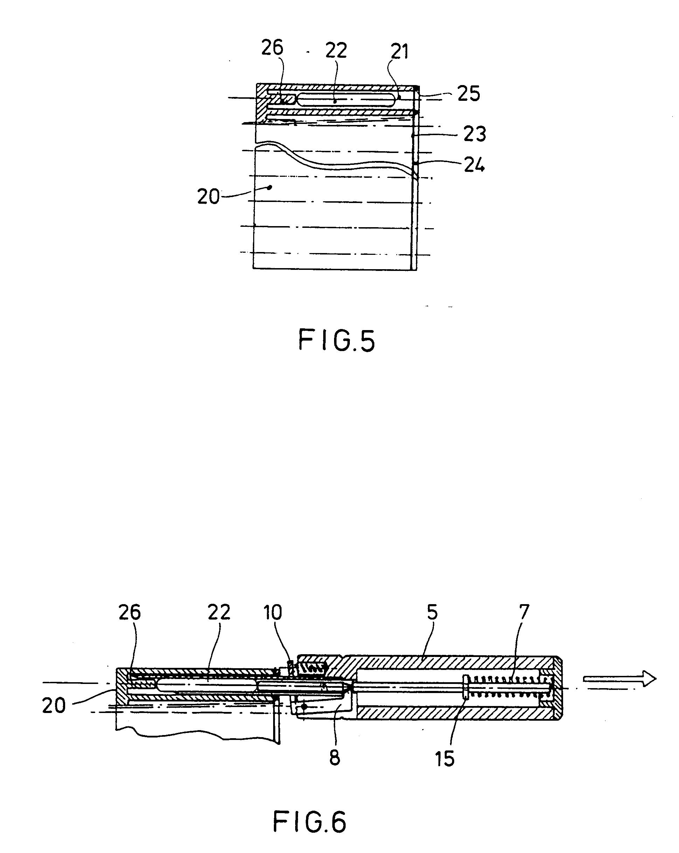

Fig. 5 schematically shows an embodiment of such a holder 20. One of the holes 21 for receiving an implant is shown in section with an implant 22 disposed therein. The hole 21 may be partly filled with a disinfectant fluid or paste. At one end the hole is closed by means of a foil 23. Provided at the other end and extending into the hole 21 is a pressure finger 26. The purpose of the pressure finger will be further explained hereinbelow.

Fig. 6 illustrates in what way the reloading of the implanter is accomplished. To that end the needle 2 of the implanter penetrates through the foil 23 into the hole 21. Upon further displacement of needle 2 into hole 21 the implant 22 is taken up into needle 2, the optional disinfectant moistening the needle internally and externally. In the process, pressure finger 26 pushes the plunger 6 into the housing 5 via the implant, thus tensioning spring 7. When the needle has almost reached the bottom of hole 21, the lock 8 engages groove 12 again. Upon retraction of the needle from hole 21 the implant is carried with it and the implanter is ready for use again. If so desired, for each use the needle can be replaced by a new one.

The exact location of the desired hole 21 by injection needle 2 can be facilitated by mounting a centering plate 24 provided with - optionally slightly conical - bores 25 guiding the injection needle in penetrating a hole 21. In a particular embodiment the foil 23 and the centering plate 24 may be formed as one whole; the foil portions 23 are then, for instance, formed as the thinner parts of a plastic centering plate 24.

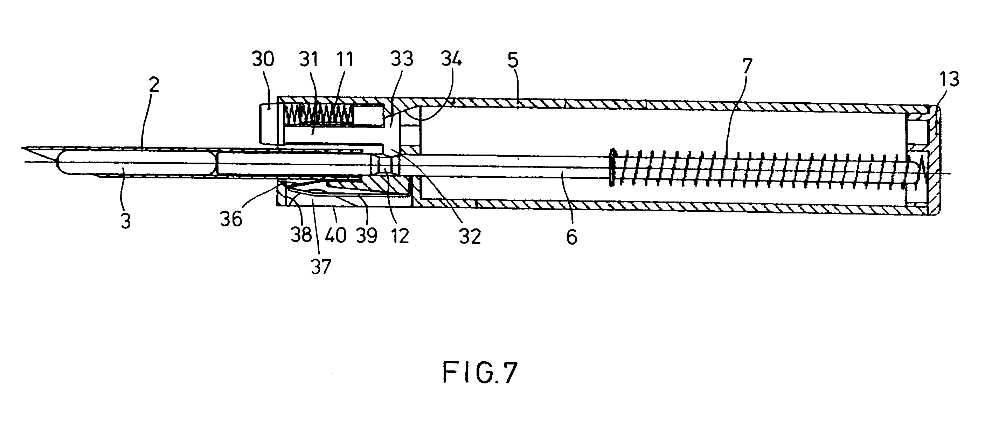

Further, Fig. 7, by way of example, shows a longitudinal sectional view of a second embodiment of an implanter according to the invention. Corresponding parts are given the same reference numerals as in Figs. 1-3. In the embodiment of Fig. 7, instead of the pivoting pressure plate 10 of Fig. 1, a pressure button 30 is used which is pressed outwardly by means of a pressure spring mounted in the housing 5. Extending rearwardly from the rear side of the pressure button is the free end of a bar or strip-shaped locking member 31 comprising at the end away from the pressure button a first projection 32 engaging when inoperative the groove 12 of the plunger 6. Although the projection 32 and the groove 12 both have bevelled edges in this embodiment, the projection is nevertheless prevented from slipping from the groove under the influence of the force exerted by the spring 7, because opposite to the projection 32 a second projection 33 is provided which rests against a projection surface 34 of the housing, this surface 34 extending obliquely inwardly towards the front of the housing.

However, the locking member 31 can move further into the housing when the pressure button 30 abuts the body of an animal and the spring 11 is compressed. Due to the fact that the projection surface extends in rearward direction precisely obliquely outwardly, the projection 33 is permitted to move slightly off the plunger 6 upon a rearward movement of the locking member 31, as a result of which the projection 32 leaves the groove 12. The plunger is then released again and will retain the implant in the manner described before while the needle is being retracted.

In the embodiment shown the needle is detachably mounted in the housing. To that end the needle comprises at least one spring element 36 operating as a barb, adapted to spring away from the needle in a chamber 37 formed in the housing, so that its front end will abut the front wall 38 of the chamber.

Further, mounted in the chamber is a needle unlocking member 39, plate-shaped in this embodiment, adapted to be pressed against the spring element 36 with the thumb. To that end a recess 40 for operating the needle unlocking member is provided. When in this manner the spring element is pressed against the needle, the needle can be pulled from the housing.

It is observed that after perusal of the above, various modifications will readily occur to a person skilled in the art. Thus,the locking member 32 may form one whole with the pressure button 30, or, conversely, be a separate element. Further, instead of the pressure springs 7 and/or 11, other types of springs can be used, such a leaf springs or extension springs.

Also, it is not necessary for the housing 5 to be closed throughout its circumference. These and similar modifications are considered to fall within the scope of the invention as defined by the appended claims.

## Claims

1. An implanter for implanting an implant in an animate being, comprising a housing (5) mounting one end of a hollow injection needle (2), spring means (7) inside the housing (5) and further a plunger (6) adapted to extend into the hollow needle (2) from the end of the hollow injection needle (2) that is turned towards the housing (5), characterized by locking means (8) for retaining the plunger against the pressure of the spring means (7) in a position where the plunger (6) extends into the hollow needle (2) over a predetermined distance, said locking means (8) comprising unlocking means (10) which abut the body of the animate being when the injection needle (2) has been introduced into the body to a predetermined depth and subsequently automatically unlock the locking means (8), causing the plunger (6) to be pressed by said spring means against an implant (3) disposed in operation in the injection needle (2) for retaining the implant (3) when the injection needle (2) is being retracted from the body.

2. An implanter according to claim 1, characterized in that the unlocking means (10) comprise a pressure member (10) provided at the side of the housing (5) where the injection needle (2) is disposed, said pressure member being mounted for rotation about a pin (9) extending transversely to the longitudinal direction of the injection needle (2) and comprising an arm (8) forming the locking means (8), one end of said arm (8) being retained under spring tension in a groove (12) in the plunger.

3. An implanter according to claim 1, characterized in that the unlocking means (30) comprise a pressure member (30) provided at the side of the housing (5) where the injection needle (2) is disposed, said pressure member (30) being movable parallel to the longitudinal direction of the injection needle (2) and cooperating with an elongate locking member (31) extending behind the pressure member (30), said locking member (31) comprising at its end away from the pressure member (30) a projection (32) cooperating with a groove (12) in the plunger (6).

4. An implanter according to claim 3, characterized in that the locking member (31) comprises a second projection (33) provided opposite to said projection (32), said second projection (33) cooperating with a projection surface (34) provided in the housing (5) and extending in the direction of the pressure member (30) and obliquely towards the injection needle (2).

5. An implanter according to claim 2, 3, or 4, characterized in that the pressure member (30) comprises a portion arranged before the housing (5).

6. An implanter according to one of claims 2-5, chracterized in that between the pressure member (30) and the housing (5) spring means (11) are operative, pressing the pressure member (30) in the direction of the tip of the injection needle (2).

7. An implanter according to one of the preceding claims, characterized in that the housing (5) at its end away from the needle (2) comprises a support member (13) and that a pressure spring (7) is mounted between the support member (13) and a shoulder formed at a distance from the end of the plunger (6) away from the injection needle (2).

8. An implanter according to claim 7, characterized in that the support member (13) is formed by a closing member closing the housing (5).

9. An implanter according to any one of the preceding claims, characterized in that the injection needle (2) comprises at least one spring barb (36) extending in mounted position in a chamber (37) of the housing (5), and that further a needle unlocking member (39) is provided adapted to be pushed against the spring barb (36) in the chamber (37) so as to push the barb (36) against the injection needle (2).

10. A loading device for an implanter according to any one of the preceding claims, characterized by a holder (20) provided with one or more holes (21) each containing an aseptic implant (22), said holes (21) being sealed on one side by means of a sealing foil (23) adapted to be pierced by the injection needle, each hole (21) having a pressure finger (26) provided internally at the end opposite the foil (23), each hole (21) having a shape such that the injection needle (2) of the implanter can be pushed into the hole (21), thus cutting through the seal (23) of the hole (21), and subsequently encloses the implant (22), during which motion spring means (7) of the implanter cooperating with the plunger (6) are tensioned via the pressure finger (26) and the implant (22), until the plunger (6) is again locked by the locking means (8), after which the implanter, upon retraction of the injection needle (2) from the reloading device, is ready again for a new implanting operation.

11. A loading device according to claim 10, characterized in that the holes (21) in the holder (20) contain a disinfectant fluid or paste in addition to an implant (22).

12. A loading device according to claim 10 or 11 and optionally 6, characterized in that a centering device (24) is mounted for guiding the injection needle (2) of the implanter into the selected implant hole (21) during loading.

13. A loading device according to one of claims 10-12,characterized in that the seal (23) of the holes (21) and the centering device (24) form one whole.

14. An implanter according to any one of claims 1-9 comprising a cap (1) protecting the injection needle (2) during transportation, said cap containing a disinfectant.

## Patentansprüche

1. Implantiervorrichtung zum Implantieren eines Implantats in ein Lebewesen, mit einem Gehäuse (5), das ein Ende einer hohlen Injektionsnadel (2), eine Federeinrichtung (7) in dem Gehäuse (5) und ferner einen Stößel (6) umfaßt, der derart ausgebildet ist, daß er sich von dem dem Gehäuse (5) zugewandten Ende der hohlen Injektionsnadel (2) in die Hohlnadel (2) erstreckt,
gekennzeichnet durch eine Verriegelungseinrichtung (8) zum Zurückhalten des Stößels gegen den Druck der Federeinrichtung (7) in einer Position, in der der Stößel (6) sich über eine vorbestimmte Länge in die Hohlnadel (2) erstreckt, wobei die Verriegelungseinrichtung (8) eine Entriegelungseinrichtung (10) aufweist, die an den Körper des Lebewesens anschlägt, wenn die Injektionsnadel (2) mit einer vorbestimmten Tiefe in den Körper eingeführt worden ist, und die anschließend die Verriegelungseinrichtung (8) automatisch entriegelt und bewirkt, daß der Stößel (6) von der Federeinrichtung gegen ein in der Injektionsnadel (2) bereitgehaltenes Implantat (3) gedrückt wird, um das Implantat (3) zurückzuhalten, wenn die Injektionsnadel (2) aus dem Körper gezogen wird.

2. Implantiervorrichtung nach Anspruch 1, dadurch gekennzeichnet, daß die Entriegelungseinrichtung (10) ein Druckteil (10) aufweist, das an derjenigen Seite des Gehäuses (5) vorgesehen ist, an der die Injektionsnadel (2) angeordnet ist, wobei das Druckteil zum Drehen um einen sich quer zu der Längsrichtung der Injektionsnadel (2) erstreckenden Stift (9) montiert ist, der einen die Verriegelungseinrichtung (8) bildenden Arm (8) aufweist, wobei ein Ende des Armes (8) unter Federspannung in einer Nut (12) in dem Stößel zurückgehalten ist.

3. Implantiervorrichtung nach Anspruch 1, dadurch gekennzeichnet, daß die Entriegelungseinrichtung (30) ein Druckteil (30) aufweist, das an derjenigen Seite des Gehäuses (5) vorgesehen ist, an der die Injektionsnadel (2) angeordnet ist, wobei das Druckteil (30) parallel zu der Längsrichtung der Injektionsnadel (2) bewegbar ist und mit einem sich hinter dem Druckteil (30) erstreckenden länglichen Verriegelungsteil (31) zusammenwirkt, wobei das Verriegelungsteil (31) an seinem dem Druckteil (30) abgewandten Ende einen mit einer Nut (12) in dem Stößel (6) zusammenwirkenden Vorsprung (32) aufweist.

4. Implantiervorrichtung nach Anspruch 3, dadurch gekennzeichnet, daß das Verriegelungsteil (31) einen gegenüber dem Vorsprung (32) vorgesehenen zweiten Vorsprung (33) aufweist, wobei der zweite Vorsprung (33) mit einer in dem Gehäuse (5) vorgesehenen und sich in der Richtung des Druckteils (30) und schräg in Richtung auf die Injektionsnadel (2) erstreckenden Vorsprungsfläche (34) zusammenwirkt.

5. Implantiervorrichtung nach Anspruch 2, 3 oder 4, dadurch gekennzeichnet, daß das Druckteil (30) einen vor dem Gehäuse (5) angeordneten Bereich aufweist.

6. Implantiervorrichtung nach einem der Ansprüche 2-5, dadurch gekennzeichnet, daß zwischen dem Druckteil (30) und dem Gehäuse (5) eine Federeinrichtung (11) derart wirksam ist, daß sie das Druckteil (30) in die Richtung der Spitze der Injektionsnadel (2) drückt.

7. Implantiervorrichtung nach einem der vorhergehenden Ansprüche, dadurch gekennzeichnet, daß das Gehäuse (5) an seinem der Nadel (2) abgewandten Ende ein Stützteil (13) aufweist, und daß eine Druckfeder (7) zwischen dem Stützteil (13) und einer im Abstand von dem der Injektionsnadel (2) abgewandten Ende des Stößels (6) ausgebildeten Schulter angebracht ist.

8. Implantiervorrichtung nach Anspruch 7, dadurch gekennzeichnet, daß das Stützteil (13) von einem das Gehäuse (5) verschließenden Verschlußteil gebildet ist.

9. Implantiervorrichtung nach einem der vorhergehenden Ansprüche, dadurch gekennzeichnet, daß die Injektionsnadel (2) mindestens einen sich in der Montageposition in einer Kammer (37) des Gehäuses (5) erstreckenden Federwiderhaken (36) aufweist, und daß ferner ein Nadelentriegelungsteil (39) vorgesehen ist, das derart ausgebildet ist, daß es derart gegen den Federwiderhaken (36) in der Kammer (37) geschoben wird, daß der Widerhaken (36) gegen die Injektionsnadel (2) geschoben wird.

10. Ladevorrichtung für eine Implantiervorrichtung nach einem der vorhergehenden Ansprüche, gekennzeichnet durch einen Halter (20), der mit einem oder mehreren Löchern (21) versehen ist, von denen jedes ein aseptisches Implantat (22) enthält, wobei die Löcher (21) an einer Seite durch eine Abdichtfolie (23) abgedichtet sind, die von der Injektionsnadel durchstechbar ist, wobei jedes Loch (21) einen innen an dem der Folie (23) gegenüberliegenden Ende vorgesehenen Druckfinger (26) aufweist, wobei jedes Loch (21) eine derartige Form aufweist, daß die Injektionsnadel (2) der Implantiervorrichtung in das Loch (21) geschoben werden kann, wodurch sie die Dichtung (23) des Lochs (21) durchtrennt und anschließend das Implantat (22) umschließt, während welcher Bewegung die mit dem Stößel (6) zusammenwirkende Federeinrichtung (7) über den Druckfinger (26) und das Implantat (22) gespannt wird, bis der Stößel (6) wieder von der Verriegelungseinrichtung (8) verriegelt wird, wonach die Implantiervorrichtung beim Zurückziehen der Injektionsnadel (2) aus der Wiederladevorrichtung wieder zu einem neuen Implantiervorgang bereit ist.

11. Ladevorrichtung nach Anspruch 10, dadurch gekennzeichnet, daß die Löcher (21) in dem Halter (20) zusätzlich zu einem Implantat (22) ein desinfizierendes Fluid oder eine solche Paste enthalten.

12. Ladevorrichtung nach Anspruch 10 oder 11 und wahlweise 6, dadurch gekennzeichnet, daß eine Zentriervorrichtung (24) zum Führen der Injektionsnadel (2) der Implantiervorrichtung in das ausgewählte Implantatloch (21) während des Ladens vorgesehen ist.

13. Ladevorrichtung nach einem der Ansprüche 10-12, dadurch gekennzeichnet, daß die Abdichtung (23) der Löcher (21) und die Zentriervorrichtung (24) ein Ganzes bilden.

14. Implantiervorrichtung nach einem der Ansprüche 1-9, mit einer Kappe (1) zum Schutz der Injektionsnadel (2) während des Transports, wobei die Kappe ein Desinfektionsmittel enthält.

## Revendications

1. Dispositif d'implantation pour implanter un implant dans un être vivant comprenant un logement (5) supportant à une extrémité une aiguille d'injection creuse (2), un moyen de ressort (7) à l'intérieur du logement (5) et qui comprend de plus un piston prévu pour se développer dans l'aiguille creuse (2) à partir de l'extrémité de l'aiguille d'injection creuse (2) qui est tournée vers le logement (5), caractérisé par un moyen de blocage (8) pour retenir le piston contre la pression du moyen de ressort (7) dans une position où le piston (6) s'étend dans l'aiguille creuse (2) sur une distance prédéterminée, ledit moyen de blocage (8) comprenant un moyen de déblocage (10) qui vient en butée contre le corps de l'être vivant lorsque l'aiguille d'injection (2) a été introduite dans le corps à une profondeur prédéterminée et débloque ultérieurement automatiquement le moyen de blocage (8), amenant le piston (6) à être pressé par ledit moyen de ressort contre un implant (3) disposé pendant l'opération dans l'aiguille d'injection (2) pour retenir l'implant (3) lorsque l'aiguille d'injection (2) est rétractée du corps.

2. Dispositif d'implantation selon la revendication 1, caractérisé en ce que le moyen de déblocage (10) comprend un élément de pression (10) prévu sur le côté du logement (5) lorsque l'aiguille d'injection (2) est jetée, ledit élément de pression étant monté pour rotation autour d'une broche (9) s'étendant transversalement à la direction longitudinale de l'aiguille d'injection (2) et comprenant un bras (8) formant le moyen de blocage (8), une extrémité dudit bras (8) étant retenue sous la tension du ressort dans une rainure (12) dans le piston.

3. Dispositif d'implantation selon la revendication 1, caractérisé en ce que le moyen de déblocage (3) comprend un élément de pression (30) prévu sur le côté du logement (5) sur lequel l'aiguille d'injection (2) est monté ledit élément de pression (30) étant déplaçable parallèle à la direction longitudinale de l'aiguille d'injection (2) et coopérant avec un élément de blocage allongé (31) s'étendant derrière l'élément de pression (30), ledit élément de blocage (31) comprenant à son extrémité opposée à l'élément de pression (30) une saillie (32) coopérant avec une rainure (12) dans le piston (6).

4. Dispositif d'implantation selon la revendication 3, caractérisé en ce que le moyen de blocage (31) comprend une seconde saillie (33) prévue opposée à ladite saillie (32), ladite seconde saillie (33) coopérant avec une surface dépassante (34) prévue dans le logement (5) et s'étendant dans la direction de l'élément de pression (30) et obliquement vers l'aiguille d'injection (2).

5. Dispositif d'implantation selon la revendication 2, 3 ou 4, caractérisé en ce que l'élément de pression (30) comprend une partie disposée devant le logement (5).

6. Dispositif d'implantation selon l'une des revendications 2 à 5, caractérisé en ce qu'entre l'élément de pression (30) et le logement (5), des moyens de ressort (11) sont fonctionnels, pressant l'élément de pression (30) dans la direction de la pointe de l'aiguille d'injection (2).

7. Dispositif d'implantation selon l'une quelconque des revendications précédentes, caractérisé en ce que le logement (5), à son extrémité opposée à l'aiguille (2) comprend un élément de support (13) et en ce qu'un ressort de pression (2) est monté entre l'élément de support (13) et un épaulement formé à une certaine distance de l'extrémité du piston (6) opposée à l'aiguille d'injection (2).

8. Dispositif d'implantation selon la revendication 7, caractérisé en ce que l'élément de support (13) est formé par un élément de fermeture fermant le logement (5).

9. Dispositif d'implantation selon l'une quelconque des revendications précédentes, caractérisé en ce que l'aiguille d'injection (2) comprend au moins une barbe de ressort (36) s'étendant en position montée dans une chambre (37) du logement (5) et en ce que de plus un élément de déblocage d'aiguille (39) est prévu , destiné à être poussé contre la barbe du ressort (36) dans la chambre (37) de façon à pousser la barbe (36) contre l'aiguille d'injection (2).

10. Dispositif de chargement pour un dispositif d'implantation selon l'une quelconque des revendications précédentes, caractérisé par un porte-implants (20) prévu avec un ou plusieurs trous (21) chacun contenant un implant aseptique (22), lesdits trous (21) étant fermés sur un côté au moyen d'une feuille d'étanchéité (23) prévue pour être percée par l'aiguille d'injection, chaque trou (21) ayant un doigt de pression (26) prévu de manière interne à l'extrémité opposée de la feuille (23), chaque trou (21) ayant une forme telle que l'aiguille d'injection (2) du dispositif d'implantation peut être poussée dans le trou (21), passant ainsi à travers la feuille (23) placée sur le trou (21), et entoure ensuite l'implant (22), pendant ce mouvement les moyens de ressort (7) du dispositif d'implantation coopérant avec le piston (6) sont mis sous tension par l'intermédiaire du doigt de pression (26) et de l'implant (22) jusqu'à ce que le piston (6) soit de nouveau bloqué par le moyen de blocage (8), après quoi le dispositif d'implantation, sur rétraction de l'aiguille d'injection (2) du dispositif de rechargement, est de nouveau prêt pour une nouvelle opération d'implantation.

11. Dispositif de chargement selon la revendication 10, caractérisé en ce que les trous (21) dans le porte-implants (20) contiennent un fluide désinfectant ou pâte désinfectante en plus d'un implant (22).

12. Dispositif de chargement selon la revendication 10 ou 11 et optionnellement 6, caractérisé en ce qu'un dispositif de centrage (24) est monté pour guider l'aiguille d'injection (2) du dispositif d'implantation dans le trou d'implant sélectionné (21) pendant le chargement.

13. Dispositif de chargement selon l'une quelconque des revendications 10 à 12, caractérisé en ce que le joint (23) des trous (21) et le dispositif de centrage (24) forment un tout.

14. Dispositif d'implantation selon l'une quelconque des revendications 1 à 9, comprenant un chapeau (1) protégeant l'aiguille d'injection (2) pendant le transport, ledit chapeau contenant un désinfectant.
